# EUROPEAN PATENT SPECIFICATION

(11) **EP 4 076 385 B1**
(45) Date of publication and mention of the grant of the patent: **10.12.2025**
(21) Application number: 20838010.5
(22) Date of filing: 18.12.2020
(51) Int. Cl.: A61K 9/00, A61K 39/395, A61K 47/26, A61K 47/12, A61K 47/18, C07K 16/28

(54) **FORMULATIONS OF ANTI-PD1 ANTIBODIES**
FORMULIERUNGEN VON ANTI-PD1-ANTIKÖRPERN
FORMULATIONS D'ANTICORPS ANTI-PD1

(30) Priority: 20.12.2019 EP 19219049
(43) Date of publication of application: 26.10.2022
(73) Proprietor: Formycon AG, 82152 Planegg (DE)
(72) Inventor: SIGL, Rainer, 86899 Landsberg am Lech (DE); SCHOTT, Katharina Maria, 82152 Martinsried/Planegg (DE)
(74) Representative: Maiwald GmbH
(86) International application number: PCT/EP2020/087071
(87) International publication number: WO 2021/123202

(56) References cited:
- EP-B1- 2 691 112
- WO-A1-2015/026634
- WO-A1-2016/168716
- WO-A1-2017/087798
- WO-A1-2018/028383
- WO-A1-2018/071792
- WO-A1-2018/204374
- WO-A1-2019/171253
- US-A1- 2011 256 135
- CUI YANAN ET AL: "Monoclonal antibodies: formulations of marketed products and recent advances in novel delivery system", DRUG DEVELOPMENT AND INDUSTRIAL PHARMACY ENGLAND, INFORMA HEALTHCARE, US, vol. 43, no. 4, 1 April 2017 (2017-04-01), pages 519 - 530, XP009194748, ISSN: 1520-5762, DOI: 10.1080/03639045.2017.1278768

## Description

### FIELD OF THE INVENTION

The present invention relates to pharmaceutical compositions of an anti-PD1 antibody comprising a buffer, a sugar or sugar alcohol and a surfactant.

### BACKGROUND OF THE INVENTION

Programmed cell death protein 1 (PD-1) is a cell surface protein which acts as an immune checkpoint and prevents autoimmune diseases by suppressing T cell inflammatory activity. PD-1 binds two ligands, PD-L1 and PD-L2. PD-L1 overexpression has been found in several cancer types and it was found that the inhibition of the interaction between PD-1 and PD-L1 can enhance T cell responses and thereby mediate antitumor activity.

Several antibodies targeting either PD-1 or PD-L1 have been developed. Pembrolizumab, marketed under the trade name Keytruda^{®}, is a humanized IgG4 antibody which binds to PD-1 and blocks its interaction with PD-L1. It is presently authorized for the treatment of several cancer types including melanoma, non-small cell lung cancer, head and neck squamous cell carcinoma, bladder cancer and Hodgkin's lymphoma. Nivolumab, marketed under the trade name Opdivo^{®}, is a fully human IgG4 antibody which binds to PD-1 and blocks its interaction with PD-L1. It is presently authorized for the treatment of melanoma, non-small cell lung cancer and renal cell carcinoma.

WO 2012/135408 and WO 2019/160751 A2 disclose liquid and lyophilized formulations of pembrolizumab comprising a histidine buffer, polysorbate 80 and sucrose.

WO 2017/054646 A1 discloses formulations of an anti-PD1 antibody containing sodium acetate, α,α-trehalose dihydrate and polysorbate 20, pH 5.2.

WO 2018/028383 A1 describes a pharmaceutical formulation comprising an anti-PD1 antibody, citrate, histidine, mannitol, sodium chloride, edetate and polysorbate 20 or polysorbate 80, pH 5.5 to 6.5.

WO 2018/187057 A1 discloses pharmaceutical compositions comprising an anti-PD1 antibody, histidine, sucrose, proline and polysorbate 80, pH 6.0.

WO 2018/204368 A1 describes formulations of an anti-PD1 antibody comprising a buffer, a stabilizer, a non-ionic surfactant and an anti-oxidant.

WO 2019/142149 A2 discloses pharmaceutical compositions comprising an anti-PD1 antibody, histidine, sucrose, polysorbate 20 and EDTA, pH 6.5.

WO 2019/171253 A1 describes a pharmaceutical composition comprising anti-PD1 antibody, a disaccharide, a buffer, a chelating agent and a polysorbate, pH 4.5 to 5.5.

WO 2015/026634 A1 relates to combination therapies comprising an antagonist of PD-1 and the CDK inhibitor dinaciclib.

Nevertheless, there is still a need for stable formulations of anti-PD1 antibodies which are safe for administration to human patients.

### SUMMARY OF THE INVENTION

The present invention relates to a liquid pharmaceutical composition consisting of 1 mM to 50 mM citrate buffer, 100 mM to 300 mM sucrose, 0.05 mg/ml to 0.6 mg/ml polysorbate 80, 10 to 80 mg/ml pembrolizumab and water for injection and having a pH of 5.5.

In one embodiment the liquid pharmaceutical composition is for use in the treatment of cancer.

In one embodiment the cancer is melanoma, non-small cell lung cancer, Hodgkin lymphoma, urothelial carcinoma, head and neck squamous cell carcinoma, primary mediastinal large B-cell lymphoma, colorectal cancer, gastric or gastroesophageal junction adenocarcinoma or cervical cancer.

### BRIEF DESCRIPTION OF THE DRAWINGS

- Figure 1:: Melting curves of pembrolizumab by variation of sugar/ sugar alcohols as stabilizer
- Figure 2:: Melting curves of pembrolizumab by variation of trehalose concentration
- Figure 3:: Melting curves of pembrolizumab by variation of polysorbate (PS) 20 and polysorbate 80
- Figure 4:: Melting curves of pembrolizumab in citrate buffered solutions by variation of pH
- Figure 5:: Melting curves of pembrolizumab in histidine buffered solutions by variation of pH
- Figure 6:: Melting curves of pembrolizumab by variation of buffer system

### DETAILED DESCRIPTION OF THE INVENTION

The present invention is as defined in the claims.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. a cell or organism is defined to be obtainable by a specific method, this is also to be understood to disclose a cell or organism which is obtained by this method.

Where an indefinite or definite article is used when referring to a singular noun, e.g. "a", "an" or "the", this includes a plural of that noun unless something else is specifically stated.

The term "pharmaceutical composition" as used herein refers to any composition comprising a chemical substance or active ingredient which composition is intended for use in the medical cure, treatment, or prevention of disease and which is in such a form as to permit the active ingredient to be effective. In particular, a pharmaceutical composition does not contain excipients which are unacceptably toxic to a subject to which the composition is to be administered. The pharmaceutical compositions are sterile, i.e. aseptic and free from all living microorganisms and their spores. The pharmaceutical composition used in the present invention is liquid and stable.

In a "liquid composition" the pharmaceutically active agent, e.g. the anti-PD1 antibody, can be combined with a variety of excipients to ensure a stable active medication following storage. In one embodiment, the liquid pharmaceutical composition used in the invention is at no point lyophilized, i.e. the production method does not contain a lyophilization step and the composition is not lyophilized for storage. Liquid compositions can be stored in vials, IV bags, ampoules, cartridges, and prefilled or ready-to-use syringes.

In another embodiment which is not according to the claimed invention, the liquid composition is lyophilized after its preparation. The terms "lyophilization", "lyophilized" and "freeze-dried" refer to a process in which the material to be dried is first frozen and then the ice or frozen solvent is removed by sublimation in a vacuum environment. Preferably, the lyophilized formulation is prepared by lyophilizing the liquid pharmaceutical composition of the present invention. The skilled person is aware of protocols for lyophilization.

A "stable" liquid composition is one in which the anti-PD1 antibody contained therein essentially retains its physical stability and/or chemical stability and/or biological activity upon storage for a certain period. Preferably, the composition essentially retains upon storage its physical and chemical stability, as well as its biological activity. Various analytical techniques for measuring protein stability are available in the art and are reviewed, for example, in Peptide and Protein Drug Delivery, 247-301, Vincent Lee Ed, Marcel Dekker, Inc, New York, New York, Pubs (1991) and Jones, Adv Drug Delivery Rev, 1993, 10:29-90. For example, stability can be measured at a selected temperature for a selected time period. Stability can be evaluated qualitatively and/or quantitatively in a variety of different ways, including evaluation of aggregate formation (for example using size exclusion chromatography, by measuring turbidity, and/or by visual inspection), by assessing charge heterogeneity using cation exchange chromatography or capillary zone electrophoresis, amino-terminal or carboxy-terminal sequence analysis, mass spectrometric analysis, SDS-PAGE analysis to detect aggregated or fragmented molecules, peptide map (for example tryptic or LYS-C) analysis, evaluating biological activity or binding of the antagonist, etc.

Preferably, the pharmaceutical composition is stable at a temperature of about 40°C for at least 1 to 2 weeks, and/or is stable at a temperature of about 5°C for at least 3 months, preferably 6 months and more preferably one year, and/or is stable at a temperature of about 25°C for at least two weeks or one month. Furthermore, the formulation is preferably stable following freezing (to, e.g., -20°C) and thawing of the formulation at 25°C as described in the examples herein, for example following 1, 2, 3 or 4 cycles of freezing and thawing.

For example, in the pharmaceutical composition used in the present invention the percentage of high molecular weight species relative to the total amount of the anti-PD1 antibody as measured by size exclusion chromatography is not more than 10%, preferably not more than 5%,

A "buffer" is an aqueous solution consisting of a mixture of a weak acid and its conjugate base or vice versa which resists changes in its pH and therefore keeps the pH at a nearly constant value. The buffer of the present invention has a pH of 5.5.

The buffer used in the present invention is a citrate buffer. The citrate buffer is the only buffer present in the liquid formulation of the present invention. In particular, the liquid formulation of the present invention does not comprise a mixture of a histidine buffer and a citrate buffer.

The terms "histidine-containing buffer" and "histidine buffer" are used interchangeably herein and refer to a buffer comprising histidine. Examples of histidine buffers include histidine chloride, histidine hydrochloride, histidine acetate, histidine phosphate, and histidine sulphate. The histidine buffer of the disclosure further comprises L-histidine. The histidine buffer of the disclosure comprises histidine hydrochloride or it comprises histidine hydrochloride and L-histidine. The histidine buffer or histidine hydrochloride buffer or histidine hydrochloride/L-histidine buffer may have a pH in the range from about 5.4 to 6.0, or from about 5.5 to 5.9, or a pH of about 5.5 or 5.9.

The histidine-containing buffer may comprise histidine hydrochloride/L-histidine in a concentration of 5 to 30 mM, or of 7 to 20 mM, or of 8 to 15 mM and or of 10 mM.

The buffer may be histidine hydrochloride/L-histidine with a concentration of 10 mM and with a pH of 5.5 or 5.9.

The buffer is a citrate buffer. The citrate buffer is prepared by mixing citric acid with a citrate salt such as sodium citrate. According to the invention, the citrate buffer has a concentration of 1 mM to 50 mM, preferably of 3 mM to 40 mM, more preferably of 5 mM to 30 mM, even more preferably of 7 mM to 20 mM and most preferably of 10 mM. The citrate buffer has a pH of 5.5.

In one embodiment, the citrate buffer comprises citric acid and sodium citrate in a concentration of 10 mM. In another embodiment the citrate buffer comprises citric acid and sodium citrate in a concentration of 10 mM and with a pH of 5.5.

A "surfactant" as used herein refers to an amphiphilic compound, i.e. a compound containing both hydrophobic groups and hydrophilic groups which lowers the surface tension (or interfacial tension) between two liquids or between a liquid and a solid. A "non-ionic surfactant" has no charged groups in its head. The formation of insoluble particles during freeze/thaw cycles of antibody-containing compositions can be remarkably inhibited by addition of surfactants. Examples of "non-ionic surfactants" include e.g. polyoxyethylene glycol alkyl ethers, such as octaethylene glycol monododecyl ether, pentaethylene glycol monododecyl ether; polyoxypropylene glycol alkyl ethers; glucoside alkyl ethers, such as decyl glucoside, lauryl glucoside, octyl glucoside; polyoxyethylene glycol octylphenol ethers, such as triton X-100; polyoxyethylene glycol alkylphenol ethers, such as nonoxynol-9; glycerol alkyl esters, such as glyceryl laurate; polyoxyethylene glycol sorbitan alkyl esters, such as polysorbate; sorbitan alkyl esters, such as spans; cocamide MEA, cocamide DEA, dodecyldimethylamine oxide; block copolymers of polyethylene glycol and polypropylene glycol, such as poloxamers; and polyethoxylated tallow amine (POEA). The pharmaceutical compositions of the present disclosure can contain one or more of these surfactants in combination. The pharmaceutical compositions of the present invention contain only one non-ionic surfactant.

Non-ionic surfactants for use in the pharmaceutical compositions of the present disclosure are polysorbates such as polysorbate 20, 40, 60 or 80, and especially polysorbate 20 (i.e. Tween 20) or polysorbate 80 (i.e. Tween 80).

The concentration of the non-ionic surfactant is in the range of 0.005 to 0.06% (w/v), or in the range of 0.008 to 0.05% (w/v), or in the range of 0.01 to 0.04% (w/v), relative to the total volume of the composition.

The non-ionic surfactant may be polysorbate 20. The non-ionic surfactant may be polysorbate 20 with a concentration in the range of 0.05 to 0.6 mg/ml, or in the range of 0.08 to 0.5 mg/ml, or in the range of 0.1 to 0.4 mg/ml.

According to the invention, the non-ionic surfactant is polysorbate 80 with a concentration in the range of 0.05 to 0.6 mg/ml, preferably in the range of 0.08 to 0.5 mg/ml, more preferably in the range of 0.1 to 0.4 mg/ml and most preferably of 0.2 mg/ml.

In a particularly preferred embodiment, the non-ionic surfactant is polysorbate 80 with a concentration of 0.2 mg/ml.

The non-ionic surfactant may be polysorbate 20 with a concentration of 0.1 mg/ml.

The non-ionic surfactant may be polysorbate 20 with a concentration of 0.2 mg/ml.

The non-ionic surfactant may be polysorbate 20 with a concentration of 0.4 mg/ml.

The term "sugar" refers to an organic compound comprising only carbon, hydrogen, and oxygen, usually with a hydrogen:oxygen atom ratio of 2:1 and the empirical formula Cm(H2O)n. The term "sugar" includes mono-, di-, oligo- and polysaccharides. Examples of sugars include glucose, fructose, galactose, xylose, ribose, sucrose, mannose, lactose, maltose, trehalose, starch, and glycogen. The sugar may be a non-reducing sugar. Non-reducing sugars are sugars which are not able to act as a reducing agent, as they do not comprise a free aldehyde or ketone group. The non-reducing sugar may be selected from sucrose and trehalose.

The sugar may be trehalose. Trehalose is a non-reducing sugar. It is a disaccharide formed by a 1,1-glycosidic bond between a glucose and a fructose unit. The dihydrate form of trehalose may be used. The concentration of trehalose dihydrate in the liquid pharmaceutical composition of the present disclosure may be 100 mM to 300 mM, or 120 mM to 280 mM, or 150 mM to 250 mM or the concentration of trehalose dihydrate is 205 mM.

The sugar is sucrose. Sucrose is a non-reducing sugar. It is a disaccharide formed by a 1,2-glycosidic bond between two α-glucose units. According to the invention, the concentration of sucrose in the liquid pharmaceutical composition of the present invention is 100 mM to 300 mM, preferably the concentration of sucrose is 120 mM to 280 mM, more preferably the concentration of sucrose is 150 mM to 250 mM and most preferably the concentration of sucrose is 205 mM. A sucrose concentration of 205 mM equals to 70 mg/ml sucrose.

The liquid pharmaceutical composition of the present disclosure may comprise a sugar alcohol. Sugar alcohols are organic compounds derived from a sugar which contain a hydroxyl group attached to each carbon atom. Suitable sugar alcohols include glycerol, mannitol, sorbitol, and xylitol. The sugar alcohol may be mannitol or sorbitol. The concentration of mannitol or sorbitol in the liquid pharmaceutical composition of the present disclosure may be 100 mM to 300 mM, or 120 mM to 280 mM, or 150 mM to 250 mM or the concentration of mannitol or sorbitol may be 205 mM. The liquid pharmaceutical composition of the present disclosure may comprise 205 mM mannitol.

The liquid pharmaceutical composition of the present invention does not contain sodium chloride. The liquid pharmaceutical composition of the present invention does not contain any sodium salt. The liquid pharmaceutical composition of the present invention does not contain any inorganic salt. As used herein, an "inorganic salt" refers to an ionic compound which has osmoregulatory properties. An inorganic salt such as sodium chloride (NaCl) can dissociate in solution into its constituent ions, i.e. NaCl dissociates into Na+ and Cl- ions, which both affect the osmotic pressure, i.e. the osmolality, of the solution. Exemplary inorganic salts which are not present in the liquid pharmaceutical composition of the present invention are potassium chloride, calcium chloride, sodium chloride, sodium phosphate, potassium phosphate and sodium bicarbonate.

The liquid pharmaceutical composition of the present invention does not contain EDTA. The liquid pharmaceutical composition of the present invention does not contain pentetic acid. The liquid pharmaceutical composition of the present invention does not contain EDTA and pentetic acid. The liquid pharmaceutical composition of the present invention does not contain any chelating agent. Chelating agents can form at least one bond with a metal atom. A chelating agent is typically a multidentate ligand that can be used in compositions as a stabilizer to complex with species, which might otherwise promote instability. Exemplary chelating agents include aminopolycarboxylic acids, hydroxyaminocarboxylic acids, N-substituted glycines, 2- (2-am ino-2-oxocthyl) aminoethane sulfonic acid (BES), deferoxamine (DEF), niacinamide, desoxycholates, ethylenediaminetetraacetic acid (EDTA), diethylenetriaminepentaacetic acid (DTPA), nitrilotriacetic acid (NTA), N-2-acetamido-2-iminodiacetic acid (ADA), bis(am inoethyl)glycolether, N,N,N',N'-tetraacetic acid (EGTA), trans- diaminocyclohexane tetraacetic acid (DCTA), N- hydroxyethyliminodiacetic acid (HIMDA), N,N-bis-hydroxyethylglycine (bicine), N- (trishydroxymethylmethyl) glycine (tricine), glycylglycine, sodium desoxycholate, ethylenediamine; propylenediamine; diethylenetriamine; triethylenetetraamine (trien), ethylenediaminetetraaceto EDTA; disodium EDTA, EDTA, calcium EDTA oxalic acid and malate. In the present invention histidine and citrate are not considered as chelating agents.

The liquid pharmaceutical composition of the present invention does not contain methionine. The liquid pharmaceutical composition of the present invention does not contain L-methionine or L-methionine-HCl. The liquid pharmaceutical composition of the present invention does not contain any anti-oxidant. In the present disclosure histidine is not considered as anti-oxidant. Anti-oxidants are compounds that inhibit oxidation by reacting with oxidizing agents.

The liquid pharmaceutical composition of the present invention does not contain proline. The liquid pharmaceutical composition of the present invention does not contain glycine. The liquid pharmaceutical composition of the present invention does not contain arginine. The liquid pharmaceutical composition of the present invention does not contain glutamic acid. The liquid pharmaceutical composition of the present invention does not contain serine. The liquid pharmaceutical composition of the present invention does not contain tyrosine. The liquid pharmaceutical composition of the present invention does not contain tryptophan. The liquid pharmaceutical composition of the present invention does not contain leucine. The liquid pharmaceutical composition of the present invention does not contain phenylalanine. The liquid pharmaceutical composition of the present invention does not contain threonine. The liquid pharmaceutical composition of the present invention does not contain lysine. The liquid pharmaceutical composition of the present invention does not contain aspartate. The liquid pharmaceutical composition of the present invention does not contain asparagine. The liquid pharmaceutical composition of the present invention does not contain glutamine. The liquid pharmaceutical composition of the present invention does not contain alanineThe liquid pharmaceutical composition of the present invention does not contain cysteine. The liquid pharmaceutical composition of the present invention does not contain isoleucine. The liquid pharmaceutical composition of the present invention does not contain valine.

The liquid pharmaceutical composition of the present invention does not contain EDTA and arginine. The liquid pharmaceutical composition of the present invention does not contain EDTA and proline. The liquid pharmaceutical composition of the present invention does not contain DTPA and methionine. The liquid pharmaceutical composition of the present invention does not contain arginine and methionine. The liquid pharmaceutical composition of the present invention does not contain arginine and proline. The liquid pharmaceutical composition of the present invention does not contain any chelating agent and arginine.

The term "antibody" or "immunoglobulin" is used herein in the broadest sense and includes full length antibodies, genetically engineered antibodies, recombinant antibodies, multivalent antibodies, monoclonal antibodies, polyclonal antibodies, bispecific antibodies, multispecific antibodies, chimeric antibodies, humanized antibodies, fully human antibodies, as well as fragments of such antibodies as long as they remain functional and exhibit the desired biological activity. The "biological activity" of an antibody refers to the ability of the antibody to bind to antigen and result in a biological response which can be measured in vitro or in vivo.

A full length antibody comprises an antigen-binding variable region of the light (VL) and heavy chain (VH), a light chain constant region (CL) and heavy chain constant domains CH1, CH2 and CH3.

The term "antibody fragment" or "antigen-binding fragment" is used herein in the broadest sense and comprises a portion of a full length antibody, preferably comprising the antigen-binding or variable region thereof. An antibody fragment retains the original specificity of the parent immunoglobulin. Examples of antibody fragments include, e.g., Fab, Fab', F(ab')2, and Fv fragments, diabodies, linear antibodies, single-chain antibody molecules, and multispecific antibodies formed from antibody fragment(s).

A "monoclonal antibody" is an antibody that is specific for a single epitope of an antigen, i.e. directed against a single determinant on an antigen. Methods for producing monoclonal antibodies are known to the person skilled in the art.

The term "recombinant antibody" refers to all antibodies prepared, expressed, created or isolated by recombinant means, such as antibodies isolated from a transgenic host cell, such as e.g. a NS0 or CHO cell, or from an animal transgenic for immunoglobulin genes, or antibodies expressed using recombinant expression vectors transfected into a host cell, such as e.g. SP 2/0 mouse myeloma cells.

A "humanized antibody" is a human antibody wherein the antigen binding portion (CDR) is derived from non-human species, such as a mouse, and thus has a different specificity compared to the parent immunoglobulin. The CDR protein sequences can be modified to increase their similarities to antibody variants produced naturally in humans.

A "fully human antibody" is an antibody wherein all parts of the antibody including the antigen binding portion (CDR) are derived from human.

The term "anti-PD1 antibody" refers to an antibody that specifically binds to cell death protein 1 (PD-1) and inhibits the binding of PD-1 to its ligand PD-L1 and optionally inhibits the binding of PD-1 to its ligands PD-L1 and PD-L2. The anti-PD1 antibody thereby abolishes the suppressive effect of the PD-1/PD-L1 interaction on T cells. Known anti-PD1 antibodies include, but are not limited to, pembrolizumab, nivolumab, cemiplimab and cetrelimab.

Pembrolizumab (also known as MK-3475, SCH 900475 and lambrolizumab) is a humanized IgG4 mAb with the structure described in WHO Drug Information, Vol. 27, No. 2, pages 161-162 (2013) and which comprises the heavy and light chain amino acid sequences and CDRs described in Table 2 of WO 2018/204368. Pembrolizumab has been approved for the treatment of patients with unresectable or metastatic melanoma and for the treatment of certain patients with recurrent or metastatic head and neck squamous cell cancer (HNSCC), classical Hodgkin lymphoma (cHL), urothelial carcinoma, gastric cancer, microsatellite instability-high (MSI-H) cancer and non- small cell lung cancer. The present commercial pembrolizumab formulation contains 10 mM histidine, 70 mg/ml sucrose, 0.2 mg/ml polysorbate 80 and water for injection, pH 5.5 and is supplied in a concentration of 25 mg/ml.

Nivolumab (also known as ONO-4538, BMS-936558, MDX1106) is a fully human monoclonal IgG4 antibody which comprises the heavy and light chain amino acid sequences and CDRs described in Table 2 of WO 2018/204368. Pembrolizumab has been approved for the treatment of patients with melanoma, renal carcinoma, non- small cell lung cancer and urothelial carcinoma. The present commercial nivolumab formulation contains 30 mg/ml mannitol, 0.008 mg/ml pentetic acid, 0.2 mg/ml polysorbate 80, 2.92 mg/ml sodium chloride, 5.88 mg/ml sodium citrate dihydrate, and water for injection, pH 6.0 and is supplied in a concentration of 10 mg/ml.

The liquid pharmaceutical composition does not contain an anti-LAG3 antibody.

The anti-human PD-1 antibody is the only antibody present in the liquid pharmaceutical composition. Pembrolizumab is the only antibody present in the liquid pharmaceutical composition according to the invention.

The concentration of the anti-PD1 antibody in the pharmaceutical compositions of the present invention is typically 10-80 mg/ml, preferably 15-70 mg/ml or 15-60 mg/ml, more preferably 20-50 mg/ml or 20-40 mg/ml, and most preferably 25 mg/ml.

The pharmaceutical compositions of the present invention can be used in the treatment of cancer, in particular in the treatment of melanoma, non-small cell lung cancer, Hodgkin lymphoma, urothelial carcinoma, head and neck squamous cell carcinoma, primary mediastinal large B-cell lymphoma, colorectal cancer, gastric or gastroesophageal junction adenocarcinoma or cervical cancer.

The pharmaceutical compositions of the present disclosure may contain further active agents, in particular further anti-tumor agents such as chemotherapeutics. Examples of such chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, es tram us tine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics (e.g. calicheamicin, especially calicheamicin gammall and calicheamicin phill); dynemicin, including dynemicin A; bisphosphonates, such as clodronate; an esperamicin; as well as neocarzinostatin chromophore and related chromoprotein enediyne antibiotic chromomophores), aclacinomysins, actinomycin, authramycin, azaserine, bleomycins, cactinomycin, carabicin, caminomycin, carzinophilin, chromomycins, dactinomycin, daunorubicin, detorubicin, 6-diazo-5-oxo-L-norleucine, doxorubicin (including morpholino- doxorubicin, cyanomoφholinodoxorubicin, 2-pyrrolino-doxorubicin and deoxydoxorubicin), epirubicin, esorubicin, idarubicin, marcellomycin, mitomy cins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; razoxane; rhizoxin; sizofuran; spirogermanium; tenuazonic acid; triaziquone; 2, 2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel and doxetaxel; chlorambucil; gemcitabine; 6- thioguanine; mercaptopurine; methotrexate; platinum analogs such as cisplatin and carboplatin; vinblastine; platinum; etoposide (VP-16); ifosfamide; mitoxantrone; vincristine; vinorelbine; novantrone; teniposide; edatrexate; daunomycin; aminopterin; xeloda; ibandronate; CPT-11; topoisomerase inhibitor RFS 2000; difluoromethylornithine (DMFO); retinoids such as retinoic acid; capecitabine; and pharmaceutically acceptable salts, acids or derivatives of any of the above. Also included are anti-hormonal agents that act to regulate or inhibit hormone action on tumors such as antiestrogens and selective estrogen receptor modulators (SERMs), including, for example, tamoxifen, raloxifene, droloxifene, 4-hydroxytamoxifen, trioxifene, keoxifene, LY1 17018, onapristone, and toremifene (Fareston); aromatase inhibitors that inhibit the enzyme aromatase, which regulates estrogen production in the adrenal glands, such as, for example, 4(5)-imidazoles, aminoglutethimide, megestrol acetate, exemestane, formestane, fadrozole, vorozole, letrozole, and anastrozole; and anti-androgens such as flutamide, nilutamide, bicalutamide, leuprolide, and goserelin; and pharmaceutically acceptable salts, acids or derivatives of any of the above.

In an alternative embodiment, the pharmaceutical compositions of the present invention may be administered in combination with any of the chemotherapeutics listed above, but the chemotherapeutic is present in a separate pharmaceutical composition.

In one embodiment which is not according to the claimed invention, the present disclosure relates to a liquid pharmaceutical composition comprising: 10 to 80 mg/ml of an anti-human PD1 antibody; 100 mM to 300 mM of trehalose or a sugar alcohol; 0.005 to 0.06% (w/v) of a non-ionic surfactant and 5 to 30 mM of a histidine-containing buffer, pH 5.5 to 5.9.

In one embodiment which is not according to the claimed invention, the present disclosure relates to a liquid pharmaceutical composition comprising: 10 to 80 mg/ml of an anti-human PD1 antibody; 100 mM to 300 mM of trehalose or a sugar alcohol; 0.005 to 0.06% (w/v) of polysorbate 20 and 5 to 30 mM of a histidine-containing buffer, pH 5.5 to 5.9.

In one embodiment which is not according to the claimed invention, the present disclosure relates to a liquid pharmaceutical composition comprising: 10 to 80 mg/ml of an anti-human PD1 antibody; 100 mM to 300 mM of trehalose or a sugar alcohol; 0.005 to 0.06% (w/v) of polysorbate 20 and 5 to 30 mM of a histidine-containing buffer, pH 5.5.

In one embodiment which is not according to the claimed invention, the present disclosure relates to a liquid pharmaceutical composition comprising: 10 to 80 mg/ml of an anti-human PD1 antibody; 100 mM to 300 mM of trehalose; 0.005 to 0.06% (w/v) of a non-ionic surfactant and 5 to 30 mM of a histidine-containing buffer, pH 5.5 to 5.9.

In one embodiment which is not according to the claimed invention, the present disclosure relates to a liquid pharmaceutical composition comprising: 10 to 80 mg/ml of an anti-human PD1 antibody; 100 mM to 300 mM of trehalose; 0.005 to 0.06% (w/v) of polysorbate 20 and 5 to 30 mM of a histidine-containing buffer, pH 5.5 to 5.9.

In one embodiment which is not according to the claimed invention, the present disclosure relates to a liquid pharmaceutical composition comprising: 10 to 80 mg/ml of an anti-human PD1 antibody; 100 mM to 300 mM of trehalose; 0.005 to 0.06% (w/v) of polysorbate 20 and 5 to 30 mM of a histidine-containing buffer, pH 5.5.

In one embodiment which is not according to the claimed invention, the present disclosure relates to a liquid pharmaceutical composition comprising: 25 mg/ml of an anti-human PD1 antibody; 205 mM of trehalose or a sugar alcohol; 0.01 to 0.04% (w/v) of a non-ionic surfactant and 10 mM of a histidine-containing buffer, pH 5.5 to 5.9.

In one embodiment which is not according to the claimed invention, the present disclosure relates to a liquid pharmaceutical composition comprising: 25 mg/ml of an anti-human PD1 antibody; 205 mM of trehalose or a sugar alcohol; 0.01 to 0.04% (w/v) of polysorbate 20 and 10 mM of a histidine-containing buffer, pH 5.5 to 5.9.

In one embodiment which is not according to the claimed invention, the present disclosure relates to a liquid pharmaceutical composition comprising: 25 mg/ml of an anti-human PD1 antibody; 205 mM of trehalose or a sugar alcohol; 0.01 to 0.04% (w/v) of polysorbate 20 and 10 mM of a histidine-containing buffer, pH 5.5.

In one embodiment which is not according to the claimed invention, the present disclosure relates to a liquid pharmaceutical composition comprising: 25 mg/ml of an anti-human PD1 antibody; 205 mM of trehalose; 0.01 to 0.04% (w/v) of a non-ionic surfactant and 10 mM of a histidine-containing buffer, pH 5.5 to 5.9.

In one embodiment which is not according to the claimed invention, the present disclosure relates to a liquid pharmaceutical composition comprising: 25 mg/ml of an anti-human PD1 antibody; 205 mM of trehalose; 0.01 to 0.04% (w/v) of polysorbate 20 and 10 mM of a histidine-containing buffer, pH 5.5 to 5.9.

In one embodiment which is not according to the claimed invention, the present disclosure relates to a liquid pharmaceutical composition comprising: 25 mg/ml of an anti-human PD1 antibody; 205 mM of trehalose; 0.01 to 0.04% (w/v) of polysorbate 20 and 10 mM of a histidine-containing buffer, pH 5.5.

In one embodiment which is not according to the claimed invention, the present disclosure relates to a liquid pharmaceutical composition comprising: 25 mg/ml of pembrolizumab; 205 mM of trehalose or mannitol; 0.01 to 0.04% (w/v) of polysorbate 20 or polysorbate 80 and 10 mM of L-histidine/histidine hydrochloride, pH 5.5 to 5.9.

In one embodiment which is not according to the claimed invention, the present disclosure relates to a liquid pharmaceutical composition comprising: 25 mg/ml of pembrolizumab; 205 mM of trehalose or mannitol; 0.01 to 0.04% (w/v) of polysorbate 20 and 10 mM of L-histidine/histidine hydrochloride, pH 5.5 to 5.9.

In one embodiment which is not according to the claimed invention, the present disclosure relates to a liquid pharmaceutical composition comprising: 25 mg/ml of pembrolizumab; 205 mM of trehalose or mannitol; 0.01 to 0.04% (w/v) of polysorbate 20 and 10 mM of L-histidine/histidine hydrochloride, pH 5.5.

In one embodiment which is not according to the claimed invention, the present disclosure relates to a liquid pharmaceutical composition comprising: 25 mg/ml of pembrolizumab; 205 mM of trehalose; 0.01 to 0.04% (w/v) of polysorbate 20 or polysorbate 80 and 10 mM of L-histidine/histidine hydrochloride, pH 5.5 to 5.9.

In one embodiment which is not according to the claimed invention, the present disclosure relates to a liquid pharmaceutical composition comprising: 25 mg/ml of pembrolizumab; 205 mM of trehalose; 0.01 to 0.04% (w/v) of polysorbate 20 and 10 mM of L-histidine/histidine hydrochloride, pH 5.5 to 5.9.

In one embodiment which is not according to the claimed invention, the present disclosure relates to a liquid pharmaceutical composition comprising: 25 mg/ml of pembrolizumab; 205 mM of trehalose; 0.01 to 0.04% (w/v) of polysorbate 20 and 10 mM of L-histidine/histidine hydrochloride, pH 5.5.

In one embodiment which is not according to the claimed invention, the present disclosure relates to a liquid pharmaceutical composition consisting of: 10 to 80 mg/ml of an anti-human PD1 antibody; 100 mM to 300 mM of trehalose or a sugar alcohol; 0.005 to 0.06% (w/v) of a non-ionic surfactant, 5 to 30 mM of a histidine-containing buffer, pH 5.5 to 5.9 and water for injection.

In one embodiment which is not according to the claimed invention, the present disclosure relates to a liquid pharmaceutical composition consisting of: 10 to 80 mg/ml of an anti-human PD1 antibody; 100 mM to 300 mM of trehalose or a sugar alcohol; 0.005 to 0.06% (w/v) of polysorbate 20, 5 to 30 mM of a histidine-containing buffer, pH 5.5 to 5.9 and water for injection.

In one embodiment which is not according to the claimed invention, the present disclosure relates to a liquid pharmaceutical composition consisting of: 10 to 80 mg/ml of an anti-human PD1 antibody; 100 mM to 300 mM of trehalose or a sugar alcohol; 0.005 to 0.06% (w/v) of polysorbate 20, 5 to 30 mM of a histidine-containing buffer, pH 5.5 and water for injection.

In one embodiment which is not according to the claimed invention, the present disclosure relates to a liquid pharmaceutical composition consisting of: 10 to 80 mg/ml of an anti-human PD1 antibody; 100 mM to 300 mM of trehalose; 0.005 to 0.06% (w/v) of a non-ionic surfactant, 5 to 30 mM of a histidine-containing buffer, pH 5.5 to 5.9 and water for injection.

In one embodiment which is not according to the claimed invention, the present disclosure relates to a liquid pharmaceutical composition consisting of: 10 to 80 mg/ml of an anti-human PD1 antibody; 100 mM to 300 mM of trehalose; 0.005 to 0.06% (w/v) of polysorbate 20, 5 to 30 mM of a histidine-containing buffer, pH 5.5 to 5.9 and water for injection.

In one embodiment which is not according to the claimed invention, the present disclosure relates to a liquid pharmaceutical composition consisting of: 10 to 80 mg/ml of an anti-human PD1 antibody; 100 mM to 300 mM of trehalose; 0.005 to 0.06% (w/v) of polysorbate 20, 5 to 30 mM of a histidine-containing buffer, pH 5.5 and water for injection.

In one embodiment which is not according to the claimed invention, the present disclosure relates to a liquid pharmaceutical composition consisting of: 25 mg/ml of an anti-human PD1 antibody; 205 mM of trehalose or a sugar alcohol; 0.01 to 0.04% (w/v) of a non-ionic surfactant, 10 mM of a histidine-containing buffer, pH 5.5 to 5.9 and water for injection.

In one embodiment which is not according to the claimed invention, the present disclosure relates to a liquid pharmaceutical composition consisting of: 25 mg/ml of an anti-human PD1 antibody; 205 mM of trehalose or a sugar alcohol; 0.01 to 0.04% (w/v) of polysorbate 20, 10 mM of a histidine-containing buffer, pH 5.5 to 5.9 and water for injection.

In one embodiment which is not according to the claimed invention, the present disclosure relates to a liquid pharmaceutical composition consisting of: 25 mg/ml of an anti-human PD1 antibody; 205 mM of trehalose or a sugar alcohol; 0.01 to 0.04% (w/v) of polysorbate 20, 10 mM of a histidine-containing buffer, pH 5.5 and water for injection.

In one embodiment which is not according to the claimed invention, the present disclosure relates to a liquid pharmaceutical composition consisting of: 25 mg/ml of an anti-human PD1 antibody; 205 mM of trehalose; 0.01 to 0.04% (w/v) of a non-ionic surfactant, 10 mM of a histidine-containing buffer, pH 5.5 to 5.9 and water for injection.

In one embodiment which is not according to the claimed invention, the present disclosure relates to a liquid pharmaceutical composition consisting of: 25 mg/ml of an anti-human PD1 antibody; 205 mM of trehalose; 0.01 to 0.04% (w/v) of polysorbate 20, 10 mM of a histidine-containing buffer, pH 5.5 to 5.9 and water for injection.

In one embodiment which is not according to the claimed invention, the present disclosure relates to a liquid pharmaceutical composition consisting of: 25 mg/ml of an anti-human PD1 antibody; 205 mM of trehalose; 0.01 to 0.04% (w/v) of polysorbate 20, 10 mM of a histidine-containing buffer, pH 5.5 and water for injection.

In one embodiment which is not according to the claimed invention, the present disclosure relates to a liquid pharmaceutical composition consisting of: 25 mg/ml of pembrolizumab; 205 mM of trehalose or mannitol; 0.01 to 0.04% (w/v) of polysorbate 20 or polysorbate 80, 10 mM of a histidine-containing buffer, pH 5.5 to 5.9 and water for injection.

In one embodiment which is not according to the claimed invention, the present disclosure relates to a liquid pharmaceutical composition consisting of: 25 mg/ml of pembrolizumab; 205 mM of trehalose or mannitol; 0.01 to 0.04% (w/v) of polysorbate 20, 10 mM of a histidine-containing buffer, pH 5.5 to 5.9 and water for injection.

In one embodiment which is not according to the claimed invention, the present disclosure relates to a liquid pharmaceutical composition consisting of: 25 mg/ml of pembrolizumab; 205 mM of trehalose or mannitol; 0.01 to 0.04% (w/v) of polysorbate 20, 10 mM of a histidine-containing buffer, pH 5.5 and water for injection.

In one embodiment which is not according to the claimed invention, the present disclosure relates to a liquid pharmaceutical composition consisting of: 25 mg/ml of pembrolizumab; 205 mM of trehalose; 0.01 to 0.04% (w/v) of polysorbate 20 or polysorbate 80, 10 mM of a histidine-containing buffer, pH 5.5 to 5.9 and water for injection.

In one embodiment which is not according to the claimed invention, the present disclosure relates to a liquid pharmaceutical composition consisting of: 25 mg/ml of pembrolizumab; 205 mM of trehalose; 0.01 to 0.04% (w/v) of polysorbate 20, 10 mM of a histidine-containing buffer, pH 5.5 to 5.9 and water for injection.

In one embodiment which is not according to the claimed invention, the present disclosure relates to a liquid pharmaceutical composition consisting of: 25 mg/ml of pembrolizumab; 205 mM of trehalose; 0.01 to 0.04% (w/v) of polysorbate 20, 10 mM of a histidine-containing buffer, pH 5.5 and water for injection.

In one embodiment which is not according to the claimed invention, the present disclosure relates to a liquid pharmaceutical composition consisting of: 25 mg/ml of pembrolizumab; 205 mM of trehalose or mannitol; 0.01 to 0.04% (w/v) of polysorbate 20 or polysorbate 80, 10 mM of L-histidine/histidine hydrochloride, pH 5.5 to 5.9 and water for injection.

In one embodiment which is not according to the claimed invention, the present disclosure relates to a liquid pharmaceutical composition consisting of: 25 mg/ml of pembrolizumab; 205 mM of trehalose or mannitol; 0.01 to 0.04% (w/v) of polysorbate 20, 10 mM of L-histidine/histidine hydrochloride, pH 5.5 to 5.9 and water for injection.

In one embodiment which is not according to the claimed invention, the present disclosure relates to a liquid pharmaceutical composition consisting of: 25 mg/ml of pembrolizumab; 205 mM of trehalose or mannitol; 0.01 to 0.04% (w/v) of polysorbate 20, 10 mM of L-histidine/histidine hydrochloride, pH 5.5 and water for injection.

In one embodiment which is not according to the claimed invention, the present disclosure relates to a liquid pharmaceutical composition consisting of: 25 mg/ml of pembrolizumab; 205 mM of trehalose; 0.01 to 0.04% (w/v) of polysorbate 20 or polysorbate 80, 10 mM of L-histidine/histidine hydrochloride, pH 5.5 to 5.9 and water for injection.

In one embodiment which is not according to the claimed invention, the present disclosure relates to a liquid pharmaceutical composition consisting of: 25 mg/ml of pembrolizumab; 205 mM of trehalose; 0.01 to 0.04% (w/v) of polysorbate 20, 10 mM of L-histidine/histidine hydrochloride, pH 5.5 to 5.9 and water for injection.

In one embodiment which is not according to the claimed invention, the present disclosure relates to a liquid pharmaceutical composition consisting of: 25 mg/ml of pembrolizumab; 205 mM of trehalose; 0.01 to 0.04% (w/v) of polysorbate 20, 10 mM of L-histidine/histidine hydrochloride, pH 5.5 and water for injection.

In one embodiment which is not according to the claimed invention, the present disclosure relates to a liquid pharmaceutical composition consisting of: 25 mg/ml of pembrolizumab; 205 mM of trehalose; 0.02% (w/v) of polysorbate 80, 10 mM of L-histidine/histidine hydrochloride, pH 5.5 and water for injection.

In one embodiment which is not according to the claimed invention, the present disclosure relates to a liquid pharmaceutical composition consisting of: 25 mg/ml of pembrolizumab; 205 mM of trehalose; 0.02% (w/v) of polysorbate 80, 10 mM of L-histidine/histidine hydrochloride, pH 5.9 and water for injection.

In one embodiment which is not according to the claimed invention, the present disclosure relates to a liquid pharmaceutical composition consisting of: 25 mg/ml of pembrolizumab; 205 mM of trehalose; 0.02% (w/v) of polysorbate 20, 10 mM of L-histidine/histidine hydrochloride, pH 5.5 and water for injection.

In one embodiment which is not according to the claimed invention, the present disclosure relates to a liquid pharmaceutical composition consisting of: 25 mg/ml of pembrolizumab; 205 mM of trehalose; 0.04% (w/v) of polysorbate 20, 10 mM of L-histidine/histidine hydrochloride, pH 5.5 and water for injection.

In one embodiment which is not according to the claimed invention, the present disclosure relates to a liquid pharmaceutical composition consisting of: 25 mg/ml of pembrolizumab; 205 mM of trehalose; 0.01% (w/v) of polysorbate 20, 10 mM of L-histidine/histidine hydrochloride, pH 5.5 and water for injection.

In one embodiment which is not according to the claimed invention, the present disclosure relates to a liquid pharmaceutical composition consisting of: 25 mg/ml of pembrolizumab; 205 mM of mannitol; 0.02% (w/v) of polysorbate 80, 10 mM of L-histidine/histidine hydrochloride, pH 5.5 and water for injection.

In one embodiment which is not according to the claimed invention, the present disclosure relates to a liquid pharmaceutical composition comprising: 10 to 80 mg/ml of an anti-human PD1 antibody; 100 mM to 300 mM of a sugar or a sugar alcohol; 0.005 to 0.06% (w/v) of a non-ionic surfactant and 1 to 50 mM of a citrate buffer, pH 5.5 to 5.9.

In one embodiment which is not according to the claimed invention, the present disclosure relates to a liquid pharmaceutical composition comprising: 25 mg/ml of an anti-human PD1 antibody; 205 mM of a sugar or a sugar alcohol; 0.01 to 0.04% (w/v) of a non-ionic surfactant and 10 mM of citrate buffer, pH 5.5 to 5.9.

In one embodiment which is not according to the claimed invention, the present disclosure relates to a liquid pharmaceutical composition comprising: 25 mg/ml of pembrolizumab; 205 mM of sucrose or trehalose; 0.02 (w/v) of polysorbate 80 and 10 mM of citrate buffer, pH 5.5 to 5.9.

In one embodiment which is not according to the claimed invention, the present disclosure relates to a liquid pharmaceutical composition consisting of: 10 to 80 mg/ml of an anti-human PD1 antibody; 100 mM to 300 mM of a sugar or a sugar alcohol; 0.005 to 0.06% (w/v) of a non-ionic surfactant, 1 to 50 mM of a citrate buffer, pH 5.5 to 5.9 and water for injection.

In one embodiment which is not according to the claimed invention, the present disclosure relates to a liquid pharmaceutical composition consisting of: 25 mg/ml of an anti-human PD1 antibody; 205 mM of sucrose or trehalose; 0.01 to 0.04% (w/v) of a non-ionic surfactant, 10 mM of a citrate buffer, pH 5.5 to 5.9 and water for injection.

In one embodiment which is not according to the claimed invention, the present disclosure relates to a liquid pharmaceutical composition consisting of: 25 mg/ml of pembrolizumab; 205 mM of trehalose; 0.01 to 0.04% (w/v) of polysorbate 80, 10 mM of a citrate buffer, pH 5.5 to 5.9 and water for injection.

In one embodiment which is not according to the claimed invention, the present disclosure relates to a liquid pharmaceutical composition consisting of: 25 mg/ml of pembrolizumab; 205 mM of trehalose; 0.02 % (w/v) of polysorbate 80, 10 mM of citrate buffer, pH 5.5 to 5.9 and water for injection.

In one embodiment, the present invention relates to a liquid pharmaceutical composition consisting of: 25 mg/ml of pembrolizumab; 205 mM of sucrose; 0.02% (w/v) of polysorbate 80, 10 mM of citrate buffer, pH 5.5 and water for injection.

In one embodiment which is not according to the claimed invention, the present disclosure relates to a liquid pharmaceutical composition consisting of: 25 mg/ml of pembrolizumab; 205 mM of trehalose; 0.02% (w/v) of polysorbate 80, 10 mM of citrate buffer, pH 5.5 and water for injection.

In one embodiment which is not according to the claimed invention, the present disclosure relates to a liquid pharmaceutical composition consisting of: 25 mg/ml of pembrolizumab; 205 mM of trehalose; 0.02% (w/v) of polysorbate 80, 10 mM of citrate buffer, pH 5.9 and water for injection.

The pharmaceutical compositions may be supplied in a vial or in a pre-filled syringe. The pharmaceutical compositions may be administered by intravenous infusion, e.g. over a period of 30 minutes or less.

While the invention has been illustrated and described in detail in the drawings and foregoing description, such illustration and description are to be considered illustrative or exemplary and not restrictive. The invention is not limited to the disclosed embodiments but is as defined by the claims. Other variations to the disclosed embodiments can be understood and effected by those skilled in the art in practicing a claimed invention, from a study of the drawings, the disclosure, and the dependent claims.

The detailed description is merely exemplary in nature and is not intended to limit application and uses. The following examples further illustrate the present invention without, however, limiting the scope of the invention thereto. Various changes and modifications can be made by those skilled in the art on the basis of the description of the invention as defined by the claims.

### EXAMPLES

In the examples, composition (h) falls within the scope of the claims, the other compositions are not according to the claimed invention.

The identification of suitable liquid pharmaceutical formulations of pembrolizumab is conducted in a 2-step procedure.

The first step of the invention (example 1) was to perform a screening of 36 different compositions for highest melting points of pembrolizumab in order to identify optimal conditions and excipients to provide stability against thermal unfolding and aggregation. Based on the result of this screening 10 formulations were selected and subjected to different thermal conditions (5°C, 25 °C/ 60 % RH, 40 °C/ 75 % RH and a freeze/ thaw study) regarding physical- and chemical modifications (see example 2).

### Example 1: Screening of 36 formulations by melting temperature

### 1. Sample preparation

Pembrolizumab from the EU marketed product Keytruda^{®} (MSD Sharp & Dohme) was transferred by 3-step-dialysis using D-Tube^{™} Dialyzer Mini (Merck Millipore, Germany) into 36 different formulations containing the following classes of excipients:
The following excipients were tested as stabilizer/ tonicity agent: sucrose, trehalose, mannitol, sorbitol and histidine (a non-sugar-based excipient). The concentrations of all these substances were varied within the formulations between 150 mM and 250 mM (for sucrose, trehalose, mannitol, sorbitol) and 10 mM to 220 mM (for histidine).

The following excipients were tested as buffering systems to adjust pH: histidine/ histidine HCl and sodium citrate/ citric acid. Concentrations of buffering systems were tested between 10 mM and 50 mM. pH values of the formulations were varied between 5.3 and 6.0.

The following excipients were tested as surfactant: Polysorbate 20 and Polysorbate 80. The concentration in this example was 0.2 mg/mL for both surfactants.

Dialyzed pembrolizumab was adjusted to 10 mg/mL. Osmolality was quantified by using an Osmomat 3000D from Gonotec GmbH, Germany. Table 1 summarizes all tested formulations of pembrolizumab.

**Table 1 Overview formulation screening**

| Formulation | Tonicity agent/ stabilizer | Buffering system | Surfactant | pH | Osmolality [mOsmol/kg] |
|---|---|---|---|---|---|
| (1) | 205 mM Sucrose | 10 mM L-Histidine | 0.2 mg/mL PS 80 | pH 5.6 | 250 |
| (2) | 205 mM Trehalose | 10 mM L-Histidine | 0.2 mg/mL PS 80 | pH 5.6 | 249 |
| (3) | 150 mM Trehalose | 10 mM L-Histidine | 0.2 mg/mL PS 80 | pH 5.6 | 184 |
| (4) | 250 mM Trehalose | 10 mM L-Histidine | 0.2 mg/mL PS 80 | pH 5.6 | 309 |
| (5) | 205 mM Mannitol | 10 mM L-Histidine | 0.2 mg/mL PS 80 | pH 5.6 | 244 |
| (6) | 150 mM Mannitol | 10 mM L-Histidine | 0.2 mg/mL PS 80 | pH 5.6 | 181 |
| (7) | 250 mM Mannitol | 10 mM L-Histidine | 0.2 mg/mL PS 80 | pH 5.6 | 292 |
| (8) | 205 mM Sorbitol | 10 mM L-Histidine | 0.2 mg/mL PS 80 | pH 5.6 | 239 |
| (9) | 150 mM Sorbitol | 10 mM L-Histidine | 0.2 mg/mL PS 80 | pH 5.6 | 179 |
| (10) | 250 mM Sorbitol | 10 mM L-Histidine | 0.2 mg/mL PS 80 | pH 5.6 | 292 |
| (11) | 205 mM Sucrose | 10 mM L-Histidine | 0.2 mg/mL PS 80 | pH 5.4 | 250 |
| (12) | 205 mM Sucrose | 10 mM L-Histidine | 0.2 mg/mL PS 80 | pH 6.0 | 244 |
| (13) | 205 mM Sucrose | 10 mM Citrate | 0.2 mg/mL PS 80 | pH 5.4 | 264 |
| (14) | 205 mM Sucrose | 10 mM Citrate | 0.2 mg/mL PS 80 | pH 5.2 | 262 |
| (15) | 205 mM Sucrose | 10 mM Citrate | 0.2 mg/mL PS 80 | pH 5.7 | 268 |
| (16) | 205 mM Sucrose | 50 mM L-Histidine | 0.2 mg/mL PS 80 | pH 5.6 | 328 |
| (17) | 205 mM Trehalose | 50 mM L-Histidine | 0.2 mg/mL PS 80 | pH 5.6 | 331 |
| (18) | 205 mM Mannitol | 50 mM L-Histidine | 0.2 mg/mL PS 80 | pH 5.6 | 314 |
| (19) | 205 mM Sorbitol | 50 mM L-Histidine | 0.2 mg/mL PS 80 | pH 5.6 | 315 |
| (20) | 150 mM Sucrose | 50 mM L-Histidine | 0.2 mg/mL PS 80 | pH 5.6 | 264 |
| (21) | 150 mM Trehalose | 50 mM L-Histidine | 0.2 mg/mL PS 80 | pH 5.6 | 273 |
| (22) | 150 mM Mannitol | 50 mM L-Histidine | 0.2 mg/mL PS 80 | pH 5.6 | 260 |
| (23) | 150 mM Sorbitol | 50 mM L-Histidine | 0.2 mg/mL PS 80 | pH 5.6 | 261 |
| (24) | 250 mM Sucrose | 50 mM L-Histidine | 0.2 mg/mL PS 80 | pH 5.6 | 400 |
| (25) | 250 mM Trehalose | 50 mM L-Histidine | 0.2 mg/mL PS 80 | pH 5.6 | 383 |
| (26) | 250 mM Mannitol | 50 mM L-Histidine | 0.2 mg/mL PS 80 | pH 5.6 | 365 |
| (27) | 250 mM Sorbitol | 50 mM L-Histidine | 0.2 mg/mL PS 80 | pH 5.6 | 362 |
| (28) | 150 mM Trehalose | 10 mM L-Histidine | 0.2 mg/mL PS 20 | pH 5.6 | 190 |
| (29) | 205 mM Trehalose | 10 mM L-Histidine | 0.2 mg/mL PS 20 | pH 5.6 | 257 |
| (30) | 250 mM Trehalose | 10 mM L-Histidine | 0.2 mg/mL PS 20 | pH 5.6 | 312 |
| (31) | 220 mM L-Histidine | N/A | 0.2 mg/mL PS 80 | pH 5.6 | 364 |
| (32) | 220 mM L-Histidine | N/A | 0.2 mg/mL PS 20 | pH 5.6 | 370 |
| (33) | 220 mM L-Histidine | N/A | 0.2 mg/mL PS 80 | pH 5.3 | 388 |
| (34) | 220 mM L-Histidine | N/A | 0.2 mg/mL PS 20 | pH 5.3 | 387 |
| (35) | 220 mM L-Histidine | N/A | 0.2 mg/mL PS 80 | pH 5.9 | 347 |
| (36) | 220 mM L-Histidine | N/A | 0.2 mg/mL PS 20 | pH 5.9 | 344 |

### 2. Melting point determination

The conformational stability is a key parameter for prediction of long-term stability of proteins and was used herein to identify formulation candidates for pembrolizumab. Melting temperatures of formulations number 1 - 36 were quantified with a Prometheus NT.48 nanoDSF instrument and PR.ThermControl-CFR software. The Prometheus instrument analyzes protein unfolding transitions based on detection of intrinsic fluorescence changes under label free conditions. It measures changes in the fluorescence of tryptophan and tyrosine residues of pembrolizumab during thermal denaturation at wavelengths of 330 nm and 350 nm. Usually, the tryptophan and tyrosine residues are located in the hydrophobic core of the protein when it is in the native state. By increasing the temperature protein's conformation changes from a native folded to an unfolded state. The temperature where this transition occurs is called melting point and is used for estimating the thermal stability of the protein. The melting point temperature Tₒₙₛₑₜ temperature is defined as the temperature at which the protein starts to denature due to changes in folding. Tₘ₁ and Tₘ₂ indicate the melting points of different domains of the antibody.

Capillaries were filled with the samples described in Table 1 (all comprising 10 mg/mL pembrolizumab) and scanned with excitation power 10%. The measurement parameters are shown in Table 2.

**Table 2: Measurement parameters**

| Parameter | Setting |
|---|---|
| Temperature slope | 1 °C/min |
| Start temperature | 25 °C |
| End temperature | 95 °C |

Melting points Tₘ and unfolding onset temperature temperatures were calculated by Software PR-ThermControl-CFR from Nanotemper. Each sample was analyzed in a duplicate measurement. Table 3 summarizes the mean of the different quality attributes:

**Table 3: Melting points and onset temperatures of the formulations of Table 1**

| Formulation number | T_{m,onset} [° C] | Tₘ₁ [° C] | Tₘ₂ [° C] |
|---|---|---|---|
| (1) | 53.4 | 64.0 | 72.5 |
| (2) | 53.6 | 64.5 | 72.6 |
| (3) | 53.1 | 64.5 | 72.4 |
| (4) | 53.9 | 64.5 | 72.8 |
| (5) | 52.9 | 63.9 | 72.4 |
| (6) | 52.6 | 63.8 | 72.2 |
| (7) | 53.1 | 63.8 | 72.4 |
| (8) | 52.9 | 63.9 | 72.3 |
| (9) | 52.8 | 63.9 | 72.3 |
| (10) | 53.2 | 64.1 | 72.4 |
| (11) | 49.9 | 62.2 | 72.3 |
| (12) | 57.6 | 66.1 | 72.1 |
| (13) | 55.0 | 65.7 | 71.2 |
| (14) | 48.7 | 64.2 | 71.3 |
| (15) | 59.2 | N/A¹⁾ | 70.7 |
| (16) | 49.1 | 61.3 | 70.9 |
| (17) | 48.9 | 61.6 | 70.9 |
| (18) | 48.6 | 61.3 | 70.7 |
| (19) | 48.3 | 61.8 | 70.6 |
| (20) | 48.6 | 61.5 | 70.6 |
| (21) | 48.7 | 61.7 | 70.7 |
| (22) | 47.9 | 61.5 | 70.5 |
| (23) | 47.9 | 61.2 | 70.4 |
| (24) | 49.5 | 62.1 | 70.9 |
| (25) | 49.4 | 61.8 | 71.1 |
| (26) | 48.6 | 61.4 | 70.8 |
| (27) | 48.6 | 61.4 | 70.8 |
| (28) | 52.5 | 64.0 | 72.4 |
| (29) | 53.3 | 64.2 | 72.6 |
| (30) | 53.2 | 64.6 | 72.8 |
| (31) | 41.9 | 58.1 | 67.2 |
| (32) | 40.9 | 58.4 | 67.2 |
| (33) | 38.5 | 55.4 | 65.3 |
| (34) | 37.9 | 55.1 | 65.1 |
| (35) | 47.4 | 61.7 | 69.1 |
| (36) | 47.4 | 61.5 | 69.0 |

| | | | |
|---|---|---|---|
| ¹⁾ Melting points of domains (Tₘ₁ and Tₘ₂) are too close to each other for a separated reporting | | | |

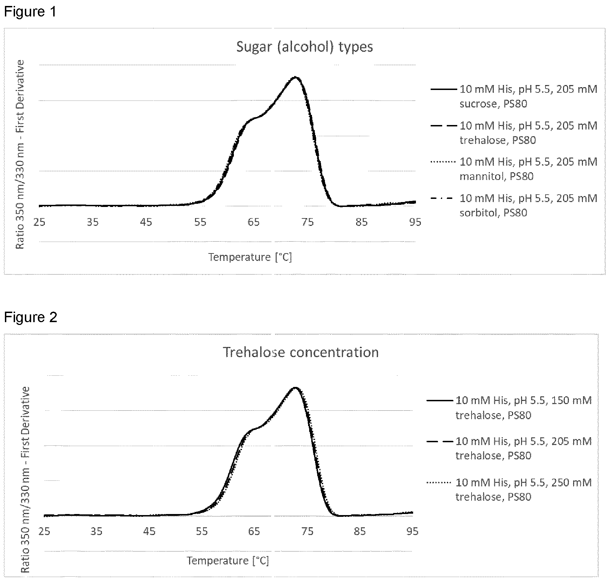

Analyzing the samples with the Prometheus system produced the following results (for detailed results see Table 3):
Surprisingly, all formulations containing sucrose, trehalose, mannitol and sorbitol (all at levels of 205 mM, in 10 mM histidine buffer, 0.2 mg/mL Polysorbate 80 at pH 5.5; i.e. formulations (1)(2)(5) and (8)) produced identical melting curves with high melting parameters (T_{m onset}, T_{m1,} Tₘ₂), which is favored for developing long term stable formulations of pembrolizumab (see figure 1). These formulations also tolerated different concentrations of the stabilizers trehalose, mannitol, sorbitol in the tested concentration range of 150 mM to 250 mM (formulations (2)(3)(4), (5)(6)(7), (8)(9)(10)) as indicated by overlapping highly identical melting behavior (see figure 2).

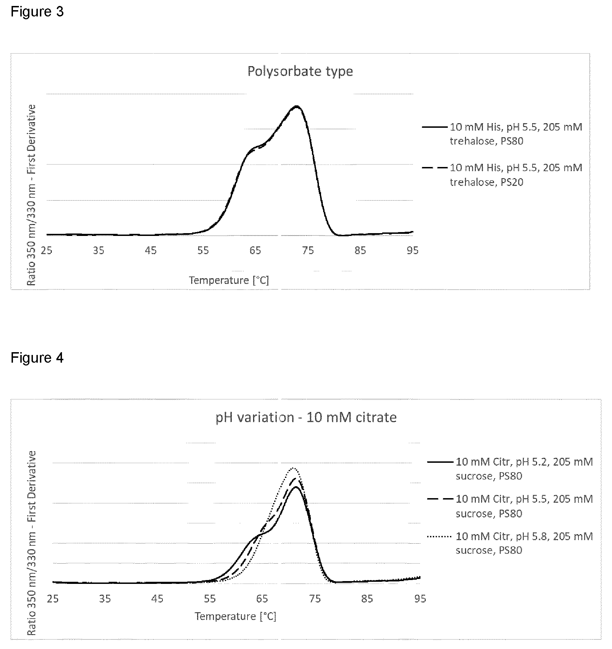

The use of the surfactants polysorbate 20 or polysorbate 80 (formulations (2)(29)) showed no difference in melting behavior as indicated by overlapping melting curves, so that these surfactants are interchangeable (see figure 3).

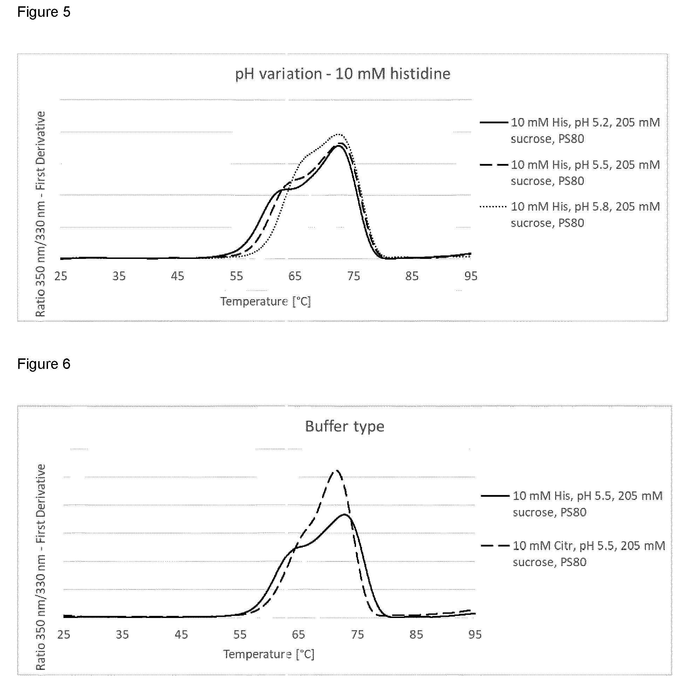

The variation of pH showed that pH 5.3 is less favored and that the higher pH formulations led to an increase of thermal stability (formulations (1)(10)(11) and (13)(14)(15); see figure 5).

When the buffer system was changed from 10 mM histidine to 10 mM citrate (formulations (1)(13)), an increase of T_{m onsets} was achieved indicating optimized thermal stability (see figure 6).

Increasing the histidine concentration to levels above 50 mM led to decreasing melting points indicating destabilizing effects on pembrolizumab. Hence, the histidine concentration should be kept below 50 mM for pembrolizumab formulations.

### Example 2

Long term-/ accelerated stability program of pembrolizumab formulations at target concentration of 25 mg/mL including a freeze/ thaw study

Pembrolizumab from the EU marketed product Keytruda^{®} (MSD Sharp & Dohme) was transferred by 3-step-dialysis using D-Tube^{™} Dialyzer (Merck Millipore, Germany) into 9 different formulations. Additionally Keytruda^{®} is included into the study as reference product.

The formulations of example 2 were chosen based on the results of example 1 and are shown in Table 4. Based on the fact that according to melting point determination both polysorbate 20 and polysorbate 80 are interchangeable, and the choice of sugar/ sugar alcohol and their range of 150 mM - 250 mM was identified as highly comparable, only a part of the potential formulations was tested in example 2. In addition to what was tested in example 1 example 2 comprises a variation of polysorbate 20 in a range of 0.1 mg/mL to 0.4 mg/mL.

**Table 4: Selected formulations**

| Formulation number | Pembrolizumab | Tonicity agent/ stabilizer | Buffering system | Surfactant | pH |
|---|---|---|---|---|---|
| (A) | 25 mg/mL | 205 mM Sucrose | 10 mM L-Histidine | 0.2 mg/mL Polysorbate 80 | pH 5.5 |
| (B) | 25 mg/mL | 205 mM Trehalose | 10 mM L-Histidine | 0.2 mg/mL Polysorbate 80 | pH 5.5 |
| (C) | 25 mg/mL | 205 mM Trehalose | 10 mM L-Histidine | 0.2 mg/mL Polysorbate 80 | pH 5.9 |
| (D) | 25 mg/mL | 205 mM Trehalose | 10 mM L-Histidine | 0.2 mg/mL Polysorbate 20 | pH 5.5 |
| (E) | 25 mg/mL | 205 mM Trehalose | 10 mM L-Histidine | 0.4 mg/mL Polysorbate 20 | pH 5.5 |
| (F) | 25 mg/mL | 205 mM Trehalose | 10 mM L-Histidine | 0.1 mg/mL Polysorbate 20 | pH 5.5 |
| (G) | 25 mg/mL | 205 mM Mannitol | 10 mM L-Histidine | 0.2 mg/mL Polysorbate 80 | pH 5.5 |
| (H) | 25 mg/mL | 205 mM Sucrose | 10 mM Citrate | 0.2 mg/mL Polysorbate 80 | pH 5.5 |
| (I) | 25 mg/mL | 205 mM Trehalose | 10 mM Citrate | 0.2 mg/mL Polysorbate 80 | pH 5.5 |
| (J) | 25 mg/mL | 205 mM Trehalose | 10 mM Citrate | 0.2 mg/mL Polysorbate 80 | pH 5.9 |

The formulations according to Table 4 containing pembrolizumab at a concentration of 25 mg/mL were filled in glass vials and stored at 5 °C for up to 24 months, at 25° C/ 60 % relative humidity for up to 6 months and at 40 °C/ 75 % relative humidity for up to 3 months. Additionally, pembrolizumab in the different formulations was stressed by five freeze/ thaw cycles. Pembrolizumab in the Keytruda^{®} formulation buffer system was included in the stability program as control sample (A).

After storage or stress treatment the samples according to Table 4 were analyzed by size exclusion chromatography (SEC) for the presence of high molecular weight species (HMWS) and by non-reducing sodium dodecyl sulfate capillary electrophoresis (CE-SDS) for the presence of fragments and HMWS. Charge heterogeneities were quantified by imaging capillary isoelectric focusing (icIEF). LC-ESI-MS and -MS/MS was used to quantify modifications like oxidation, deamidation and glycation. The activity of pembrolizumab was determined by a binding ELISA.

Additionally formulations were characterized for protein content, pH and melting point of pembrolizumab.

### Example 3

### Analysis of pembrolizumab after storage under different conditions

### 3.1 Sample preparation

Pembrolizumab from the EU marketed product Keytruda^{®} (100 mg/4 mL) was transferred by 3-step-dialysis into 10 different formulations having the composition shown in Table 5 below.

Dialyzed pembrolizumab was adjusted to 25 mg/mL ± 10 %, 0.22 µm sterile filtered and aseptically filled into 2R glass vials with a filling volume of 400 µl. The samples according to Table 5 were initially analyzed for protein concentration by UV-Vis spectroscopy at 280 nm and correct pH adjustment before starting the stability study. Afterwards samples were stored at 5°C (target storage temperature) or 25°C/ 60 % relative humidity for up to 6 months and at 40°C/ 75 % relative humidity for up to 3 months. Additionally, pembrolizumab in the different formulations was stressed by five freeze/ thaw cycles. Pembrolizumab in the Keytruda^{®} labelled formulation buffer system was included in the stability program as control sample (a).

Additionally the reference product Keytruda^{®} was characterized without any preparation step to exclude an influence of the sample handling on stability results. Analytical results confirm that pembrolizumab sourced from Keytruda^{®} EU batch S027131 was not altered after transferring into the different formulations and comparable to the reference product Keytruda^{®}.

**Table 5: Detailed information on all formulations used in this study.**

| No. | Pembrolizumab | Buffer system | Stabilizer | Surfactant | pH |
|---|---|---|---|---|---|
| (a) | 25 mg/mL | 10 mM L-histidine/ histidine HCl | 205 mM sucrose | 0.2 mg/mL polysorbate 80 | pH 5.5 |
| (b) | 25 mg/mL | 10 mM L-histidine/ histidine HCl | 205 mM trehalose | 0.2 mg/mL polysorbate 80 | pH 5.5 |
| (c) | 25 mg/mL | 10 mM L-histidine/ histidine HCl | 205 mM trehalose | 0.2 mg/mL polysorbate 80 | pH 5.9 |
| (d) | 25 mg/mL | 10 mM L-histidine/ histidine HCl | 205 mM trehalose | 0.2 mg/mL polysorbate 20 | pH 5.5 |
| (e) | 25 mg/mL | 10 mM L-histidine/ histidine HCl | 205 mM trehalose | 0.4 mg/mL polysorbate 20 | pH 5.5 |
| (f) | 25 mg/mL | 10 mM L-histidine/ histidine HCl | 205 mM trehalose | 0.1 mg/mL polysorbate 20 | pH 5.5 |
| (g) | 25 mg/mL | 10 mM L-histidine/ histidine HCl | 205 mM mannitol | 0.2 mg/mL polysorbate 80 | pH 5.5 |
| (h) | 25 mg/mL | 10 mM citric acid/ sodium citrate | 205 mM sucrose | 0.2 mg/mL polysorbate 80 | pH 5.5 |
| (i) | 25 mg/mL | 10 mM citric acid/ sodium citrate | 205 mM trehalose | 0.2 mg/mL polysorbate 80 | pH 5.5 |
| (j) | 25 mg/mL | 10 mM citric acid/ sodium citrate | 205 mM trehalose | 0.2 mg/mL polysorbate 80 | pH 5.9 |

Protein stability was determined by size exclusion chromatography (SE-HPLC) for the presence of high molecular weight species (HMWS), by non-reduced SDS-cGE for the presence of fragments (LMWS) and HMWS. Chemical modifications like glycation, oxidation and deamidation were quantified by LC-ESI-MS and -MS/MS in reduced peptide mapping. Imaged capillary isoelectric focusing (icIEF) was used to detect modifications leading to charge heterogeneities. The activity of pembrolizumab was determined by Potency ELISA.

### 3.2 Analysis of protein content by UV-Vis and pH of formulations

All samples prepared according to Table 5 were analyzed for their protein content using a Nanodrop spectral photometer and pH using a pH meter before starting the stability study (t0). Measurements were performed in triplicates (UV-Vis) or duplicates (pH).

All samples met the acceptance criteria for formulations according to Table 5 in content (25 mg/mL ± 10 % pembrolizumab) and pH (target ± 0.1). No visible particles or changes in color were detected in all samples.

### 3.3 Analysis of high molecular weight species (HMWS) by SE-HPLC

The protein samples of the stability study were diluted with the corresponding formulation buffer to a concentration of 2.5 mg/mL and 20 µl thereof were applied onto a TSKgel G3000SWXL (Tosoh, 300 x 7.8 mm, 5 µm) column to detect high molecular weight species of pembrolizumab.

The protein was eluted by isocratic elution using 0.1 M potassium phosphate buffer with 0.25 M potassium chloride (pH 5.6) at a flow rate of 0.5 mL/min at 25°C. Eluted species were detected at a wavelength of 280 nm and displayed on a graph showing the concentration of the eluted species vs. time. The elution profile showed a main peak with the non-aggregated protein and peaks of the protein representing higher molecular weight forms of the protein. The areas of all peaks were determined.

Table 6 shows the percentage of peak area for the HMWS in relation to the total peak area of the eluted species for the samples of Table 5. Each sample was examined in duplicate measurements.

**Table 6: Percentage of HMWS in different formulations as determined via SE-HPLC**

| Condition | Formulation | HMWS [%] |
|---|---|---|
| Keytruda batch S027131 | See above | 0.15 |
| T0 | (a) | 0.14 |
| | (b) | 0.18 |
| | (c) | 0.16 |
| | (d) | 0.18 |
| | (e) | 0.18 |
| | (f) | 0.16 |
| | (g) | 0.72 |
| | (h) | 0.14 |
| | (i) | 0.15 |
| | (j) | 0.19 |
| 6M 5 °C | (a) | 0.21 |
| | (b) | 0.23 |
| | (c) | n.d. |
| | (d) | 0.19 |
| | (e) | 0.18 |
| | (f) | 0.16 |
| | (g) | n.d. |
| | (h) | 0.16 |
| | (i) | 0.13 |
| | (j) | n.d. |
| 5 Freeze/ thaw cycles | (a) | 0.12 |
| | (b) | 0.14 |
| | (c) | 0.16 |
| | (d) | 0.15 |
| | (e) | 0.14 |
| | (f) | 0.15 |
| | (g) | 7.59 |
| | (h) | 0.14 |
| | (i) | 0.19 |
| | (j) | 0.19 |

At target storage condition 5°C the generation of HMWS was lowest in formulation (i), using a citrate buffer, 205 mM trehalose and 0.2 mg/mL polysorbate 80 with a pH 5.5.

Further optimal conditions for formulations of pembrolizumab were identified in samples (h) and (f). Also here the content of HMWS was more or less constant during the full storage time of 6 months at 5°C indicating optimal conditions for stabilizing pembrolizumab against the formation of aggregates at intended storage conditions.

Comparing the histidine buffered formulations (a, b, d, e, f) containing either polysorbate 80 or polysorbate 20 at pH 5.5, an advantage could be demonstrated if polysorbate 20 was used as surfactant (formulations d, e, f). After 6 months storage at 5°C the content of HMWS in the polysorbate 20-containing histidine buffered formulations was slightly lower than in the polysorbate 80-containing histidine buffered formulations (a) and (b).

All tested formulations except formulation (g) were tested as stable against multiple freeze/ thaw cycles. The formulation (g) containing mannitol as excipient showed strong generation of HMWS after freezing and thawing.

### 3. 4. Detection of modifications by LC-ESI-MS and -MS/MS

Analysis by LC-ESI-MS and -MS/MS was used for sequencing pembrolizumab by peptide mapping. Several post-translational modifications (oxidation, deamidation and glycation) were quantified using a combination of several different digestion conditions followed by LC-ESI-MS and -MS/MS measurement. LC-ESI-MS and -MS/MS mass spectra were obtained using the UltiMate^{®} 3000 system (Thermo Fisher Scientific) coupled to a Q Exactive Orbitrap Plus mass spectrometer (Thermo Fisher Scientific). The separation of the peptides was performed with reversed phase (RP) chromatography on an Accucore RP - MS LC column (2.1 x 100 mm, 2.6 µm particle size, Thermo Fisher Scientific). The following eluents were used: A: water with 0.1 % formic acid; B: acetonitrile with 0.1 % formic acid. A segmented gradient from 3 % B to 36 % B in 45 min at 30°C with a flow rate of 0.4 mL/min was applied. MS and MS/MS spectra (produced with Higher Energy Collisional Dissociation, HCD) were recorded in positive ion mode with internal mass calibration. The datasets were searched with ProteinMetrics Byonic^{™} against a sequence database consisting of the pembrolizumab sequence and common contaminants (e.g. sequences of used proteases) and analyzed for deamidation level of asparagine/glutamine, oxidation level of methionine/tryptophan and glycation level. Samples from the same stability pull point were proteolytically digested, reduced and alkylated at the same time.

Samples shown in Table 5 were analyzed as single measurement before and after 3 months incubation at 40°C/ 75 % relative humidity. The analysis of samples after storage at 40°C for 3 months was chosen for evaluation of trends at accelerated conditions which will also occur at target storage temperature, but with slower velocity. Hence, after 6 months storage at 5°C modifications would probably not exceed method variations.

After 3 months storage at 40°C several methionine and tryptophan oxidations in pembrolizumab could be identified, wherein methionine oxidation, especially at sites M105 (optionally combined with oxidation at W110), and M252 (see Table 7) was predominant.

**Table 7: Percentage of oxidized species in different formulations**

| Condition | Formulation | Oxidation M105 [%] | Oxidation M105 W110 [%] | Oxidation M252 [%] |
|---|---|---|---|---|
| Keytruda batch S027131 | See above | 2.97 | 5.06 | 2.24 |
| T0 | (a) | 3.80 | 5.22 | 2.51 |
| | (b) | 3.51 | 5.45 | 2.57 |
| | (c) | 3.20 | 5.45 | 2.44 |
| | (d) | 3.65 | 5.52 | 2.64 |
| | (e) | 3.53 | 6.16 | 2.69 |
| | (f) | 3.57 | 6.04 | 2.87 |
| | (g) | 3.50 | 5.47 | 2.59 |
| | (h) | 3.12 | 4.98 | 2.41 |
| | (i) | 3.72 | 5.38 | 2.65 |
| | (j) | 3.94 | 5.82 | 2.47 |
| 3M 40 °C | (a) | 10.46 | 16.43 | 5.05 |
| | (b) | 10.83 | 16.29 | 4.96 |
| | (c) | 12.08 | 16.44 | 5.20 |
| | (d) | 8.32 | 15.06 | 4.72 |
| | (e) | 8.60 | 13.25 | 4.65 |
| | (f) | 9.41 | 13.82 | 4.98 |
| | (g) | 14.53 | 20.10 | 4.80 |
| | (h) | 7.61 | 10.38 | 4.11 |
| | (i) | 10.02 | 15.38 | 5.23 |
| | (j) | 10.56 | 15.19 | 5.17 |

It could be demonstrated that the lowest content of M105 oxidation was obtained when using formulation (h) which comprises a citrate buffered formulation of pembrolizumab. Comparing histidine buffered formulations (a) - (f) using either polysorbate 80 or polysorbate 20 as surfactant an advantage could be demonstrated, if polysorbate 20 was used (formulations (d), (e) and (f)) compared to the use of polysorbate 80 (formulations (a), (b) and (c)). The extent of M105 oxidation was lower in the histidine buffered formulations containing polysorbate 20 than in the polysorbate 80-containing histidine buffered formulations.

After 3 months storage at 40 °C several deamidations of asparagine and glutamine could be identified. Deamidation of N325 was predominant (see Table 8).

**Table 8: Percentage of deamidated species in different formulations**

| Condition | Formulation | Deamidation N325 [%] |
|---|---|---|
| Keytruda batch S027131 | See above | 4.29 |
| T0 | (a) | 4.60 |
| | (b) | 4.49 |
| | (c) | 6.44 |
| | (d) | 6.14 |
| | (e) | 4.32 |
| | (f) | 4.88 |
| | (g) | 3.94 |
| | (h) | 4.01 |
| | (i) | 4.21 |
| | (j) | 4.65 |
| 3M 40 °C | (a) | 68.58 |
| | (b) | 65.93 |
| | (c) | 70.64 |
| | (d) | 67.06 |
| | (e) | 69.72 |
| | (f) | 71.79 |
| | (g) | 71.82 |
| | (h) | 60.40 |
| | (i) | 72.55 |
| | (j) | 70.92 |

According to Table 8 the lowest content of N325 deamidation was obtained when using formulation (h) which comprises a citrate buffered formulation of pembrolizumab compared to all other tested formulations in which the N325 deamidation was in a comparable range.

After three months storage at 40°C several glycations of lysine could be identified and were summed up for evaluation of the total glycation (see Table 9). All glycation sites were equally distributed, no predominant sites were identified.

**Table 9: Percentage of glycated species in different formulations**

| Condition | Formulation | Total Glycation [%] |
|---|---|---|
| Keytruda batch S027131 | See above | 3.62 |
| T0 | (a) | 3.67 |
| | (b) | 3.63 |
| | (c) | 3.58 |
| | (d) | 3.62 |
| | (e) | 3.52 |
| | (f) | 2.98 |
| | (g) | 3.08 |
| | (h) | 2.95 |
| | (i) | 3.71 |
| | (j) | 3.97 |
| 3M 40 °C | (a) | 14.12 |
| | (b) | 8.93 |
| | (c) | 9.08 |
| | (d) | 9.62 |
| | (e) | 9.31 |
| | (f) | 8.62 |
| | (g) | 2.35 |
| | (h) | 13.62 |
| | (i) | 8.48 |
| | (j) | 10.61 |

It could be demonstrated that contents of glycation were lower when using trehalose (formulations (b), (c), (d), (e), (f), (i), (j)) instead of sucrose (formulations (a), (h)) as stabilizer in the formulation. The lowest content of glycation was obtained with formulation (g), which comprises mannitol as stabilizer.

### 3.5. Detection of high molecular weight species (HMWS) and low molecular weight species (LMWS) by SDS-cGE non-reduced

Capillary gel electrophoresis was carried out based on the IgG Purity and Heterogeneity Analysis established by Beckman Coulter. The samples were diluted in SDS Sample Buffer pH 6.4 - 6.9 (10 mM citrate phosphate, 1% SDS) to a final concentration of 1 mg/mL for non-reduced analysis. Afterwards the thiol alkylating reagent N-Ethylmaleimide (NEM; 10 mM) was added to the sample mix to prevent fragmentation. Prior to analysis the sample was heat denatured at 70°C for 10 min. For the cGE Method, separation was performed by forward injection in a neutral, bare fused silica capillary (20 cm effective length and 50 µm diameter) with a PA800 plus instrument from Beckman Coulter. After voltage forced application of the sample (5 kV for 20 s) into the capillary, protein separation was performed by applying a voltage of 15 kV for 30 min in case of reducing conditions and 15 kV for 40 min in case of non-reducing conditions. UV absorption was measured at 220 nm using the PDA detector and a 100 x 200 aperture. The capillary temperature was kept constant at 25°C for all steps. The autosampler temperature was set to 15°C. Data were evaluated in terms of peak integration using the 32Karat software (Beckman Coulter). Peak areas were determined as velocity-corrected relative peak areas, considering the fact that in capillary electrophoresis early peaks migrate faster through the detector window than later peaks. Sample peak integration was performed in comparison to the electropherogram of a formulation buffer or pure water blank to identify and exclude non-protein-specific peaks.

The samples shown in Table 5 were analyzed as single measurement at different time-points during incubation for 3 months at 40°C/ 75 % relative humidity and for 6 months at 5°C and 25°C/ 60 % relative humidity.

All tested formulations were stable upon storage for 6 months at target storage temperature 5°C. LMWS were just slightly increased from starting level 1.4 % - 1.5 % to 1.5 % - 1.6 %, which are excellent values promising long term stability regarding the generation of LMWS tested by SDS-cGE non-reduced. No or negligible (0.1 %) HMWS were detected in all tested formulations for this incubation period. Also by applying several freeze/thaw cycles no LMWS or HMWS were generated in all tested formulations. Storage at higher temperatures (6 months 25°C/ 60 % RH, 3 months 40°C/ 75 % RH) led to slightly increased values as expected for a storage at accelerated conditions. All tested formulations comprised LMWS and a slight increase of HMWS in a very comparable range.

### 3.6. Detection of acidic species and basic species by icIEF

The aim of imaged capillary isoelectric focusing (icIEF) is to determine the isoelectric point (pl) and the heterogeneity of charge isoforms of a protein that are caused by posttranslational modifications (PTM). The power of iclEF lies in the high resolving electropherograms allowing a precise and reproducible relative quantification of the charge isoforms.

Samples were rebuffered by ultrafiltration against ultrapure water and interfering buffer components were depleted from the samples using Detergent Removal Spin Columns (Pierce) according to the manufacturers' instructions. The protein concentration was determined by UV-measurement (absorption at 280 nm). The rebuffered sample was diluted to 1 g/L with ultrapure water. 40 µg diluted sample (corresponds to a final protein concentration of 0.2 g/L) were mixed with 2 % ampholytes (0.25 % pH 9-11 and 3 % 8-10.5), 0.35 % methylcellulose, 4 M Urea and pl markers (8.18 and 9.99). Focusing was performed in two steps (1 min at 1500 V and 10 min at 3000 V). Final analysis was carried out with the imaged CEsystem Maurice C (ProteinSimple). Data were evaluated by pl calibration of the electropherograms using the two internal pl markers and Compass for iCE software (ProteinSimple). Peak integration was performed using Empower software (Waters). Calculations with relative peak areas (e.g. for peak grouping) were performed using initial values for relative peak areas with three decimal places. The initial values used for calculations and the calculated results are reported rounded to one decimal place.

Samples shown in Table 5 were analyzed as single measurement at different time-points during incubation for 3 months at 40°C/ 75 % relative humidity and for 6 months at 5°C and 25°C/ 60 % relative humidity.

Pembrolizumab showed excellent stability in all tested formulations upon storage at 5°C for six months, as no significant changes in acidic species and basic species were quantified. During 3 months storage at 25°C/ 60% relative humidity and 40°C/ 75 % relative humidity a shift to acidic species was detected. This shift led to a decrease of main peak and basic species, but no clear variation between the tested formulations could be detected which exceeds method variation. A slightly increased loss of main peak might be seen in formulations (c), (i) and (j).

### 3.7. Determination of the relative potency of pembrolizumab

The relative potency of pembrolizumab was determined by an ELISA (Enzyme-linked immunosorbent assay). The assay principle is an indirect ELISA in which PD-1 is coated on a 96 well polystyrene plate. After a blocking step and binding of serially diluted reference and test samples to PD-1, bound pembrolizumab was detected by adding a HRP-labelled detection antibody with the subsequent use of TMB as colorimetric substrate. The development was stopped by addition of sulfuric acid and the plate was read on an ELISA plate reader at 450 nm. A 4PL-fit was applied to the data and activity of samples was determined in relation to a reference by parallel analysis and expressed as relative potency.

All tested formulations showed unaltered relative potencies of pembrolizumab when stored for 3 months at 25°C/ 60 % relative humidity or 40°C/ 75 % relative humidity. Since the results obtained with samples stored under accelerated conditions showed unaltered relative potencies, it can be postulated that potency levels are also stable upon storage at 5 °C.

Relative potency at target storage temperature 5 °C will be determined at later time-points of the stability study (e.g. after storage for 12 months).

### 3.8. Summary of stability results

Based on the results generated with the shown method set some excipients could be identified which stabilize pembrolizumab in liquid formulations and are superior compared to other excipients tested or even compared to the reference product formulation. Hence, formulations comprising these excipients are suitable for long term storage of pembrolizumab.

The most prominent effect of using trehalose as stabilizer in the formulation is the stability of the antibody against glycation. Compared to sucrose-containing formulations the generation of lysine glycation was decreased in trehalose-containing formulations. It could also be shown that trehalose is effective to stabilize pembrolizumab against aggregation at the target storage temperature 5°C.

In histidine-buffered formulations polysorbate 20 improved the stability against methionine oxidation when compared to using polysorbate 80 as surfactant. In combination with histidine and trehalose lowest levels of aggregation were quantified with SE-HPLC at target storage temperature when using polysorbate 20.

Surprisingly it was found that a formulation comprising a citrate buffer stabilized pembrolizumab against aggregation at target storage temperature 5°C, even better than histidine based formulations. Formulation (h), comprising the combination of citrate buffer with sucrose and polysorbate 80 led to promising results also in minimizing deamidation and oxidation. Although not tested so far, replacing polysorbate 80 with 20 might lead to further stability improvements based on the information from this stability study.

## Claims

1. A liquid pharmaceutical composition consisting of 1 mM to 50 mM citrate buffer, 100 mM to 300 mM sucrose, 0.05 mg/ml to 0.6 mg/ml polysorbate 80, 10 mg/ml to 80 mg/ml pembrolizumab and water for injection and having a pH of 5.5.

2. The liquid pharmaceutical composition of claim 1, consisting of 10 mM citrate buffer, 205 mM sucrose, 0.2 mg/ml polysorbate 80, 25 mg/ml pembrolizumab and water for injection and having a pH of 5.5.

3. The pharmaceutical composition of claim 1 or 2 for use in the treatment of cancer.

4. The pharmaceutical composition for use according to claim 3, wherein the cancer is melanoma, non-small cell lung cancer, Hodgkin lymphoma, urothelial carcinoma, head and neck squamous cell carcinoma, primary mediastinal large B-cell lymphoma, colorectal cancer, gastric or gastroesophageal junction adenocarcinoma or cervical cancer.

## Patentansprüche

1. Flüssige pharmazeutische Zusammensetzung, bestehend aus 1 mM bis 50 mM Citratpuffer, 100 mM bis 300 mM Saccharose, 0.05 mg/ml bis 0.6 mg/ml Polysorbat 80, 10 mg/ml bis 80 mg/ml Pembrolizumab und Wasser für Injektionszwecke, mit einem pH-Wert von 5.5.

2. Flüssige pharmazeutische Zusammensetzung nach Anspruch 1, bestehend aus 10 mM Citratpuffer, 205 mM Saccharose, 0.2 mg/ml Polysorbat 80, 25 mg/ml Pembrolizumab und Wasser für Injektionszwecke, mit einem pH-Wert von 5,5.

3. Pharmazeutische Zusammensetzung nach Anspruch 1 oder 2 zur Verwendung in der Behandlung von Krebs.

4. Pharmazeutische Zusammensetzung zur Verwendung nach Anspruch 3, wobei der Krebs Melanom, nicht-kleinzelliges Lungenkarzinom, Hodgkin-Lymphom, Urothelkarzinom, Plattenepithelkarzinom der Kopf- und Halsregion, primär mediastinales großzelliges B-Zell-Lymphom, Kolorektalkarzinom, Adenokarzinom des Magens oder des gastroösophagealen Übergangs oder ein Zervixkarzinom ist.

## Revendications

1. Composition pharmaceutique liquide composée de 1 mN à 50 mM de tampon de citrate, de 100 mM à 300 mM de sucrose, de 0,05 mg/ml à 0,6 mg/ml de polysorbate 80, de 10 mg/ml à 80 mg/ml de pembrolizumab et d'eau pour injection et ayant un pH de 5,5.

2. La composition pharmaceutique liquide de la revendication 1, composée de 10 mM de tampon de citrate, de 205 mM de sucrose, de 0,2 mg/ml de polysorbate 80, de 25 mg/ml de pembrolizumab et d'eau pour injection et ayant un pH de 5,5.

3. La composition pharmaceutique de la revendication 1 ou 2 pour utilisation dans le traitement d'un cancer.

4. La composition pharmaceutique pour utilisation selon la revendication 3, sachant que le cancer est un mélanome, un cancer du poumon non à petites cellules, un lymphome hodgkinien, un carcinome urothélial, un carcinome épidermoïde de la tête et du cou, un lymphome à grandes cellules B primitif du médiastin, un cancer colorectal, un adénocarcinome gastrique ou de la jonction cesogastrique ou un cancer du col de l'utérus.
